(19) Europäisches Patentamt
European Patent Office
Office européen des brevets

(11) EP 1 927 350 A1

(12) **EUROPEAN PATENT APPLICATION**

(43) Date of publication:
**04.06.2008 Bulletin 2008/23**

(51) Int Cl.:
***A61K 31/165*** *(2006.01)* ***A61K 45/06*** *(2006.01)*
***A61P 9/12*** *(2006.01)* ***A61P 9/00*** *(2006.01)*

(21) Application number: **06124774.8**

(22) Date of filing: **24.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Novartis AG**
**4056 Basel (CH)**

(72) Inventor: **The designation of the inventor has not yet been filed**

(74) Representative: **Hillebrand, Dirk**
**Novartis AG**
**Corparate Intellectual Property**
**4002 Basel (CH)**

(54) **Methods for improving bioavailability of a renin inhibitor**

(57) The present invention provides a method for improving the bioavailability of a renin inhibitor, preferably, of a δ-amino-γ-hydroxy-ω-aryl-alkanoic acid derivative, which method comprises co-administering to a mammal, especially a human, in need of such treatment, a combination of a renin inhibitor, or a pharmaceutically acceptable salt thereof, and an MDR1 inhibitor selected from a non-pharmacologically active compound.

EP 1 927 350 A1

## Description

The oral route is often the most convenient route for drug administration, but unfortunately many therapeutic agents are not orally active due to their poor bioavailability.

The bioavailability of many therapeutic agents may be reduced by the action of so-called "efflux pump" proteins which actively eject foreign substances from the cell to give rise, for example, to the multidrug resistance effect. These drug efflux proteins principally comprise MDR (multidrug resistance protein) and MRP (multidrug resistance associated protein) type transporters. Some of the best studied efflux proteins include P-glycoprotein (Pgp or MDR1) and MRP2.

Although membrane located efflux proteins are well known as a factors contributing to the acquired multidrug resistance syndrome arising in many cancer patients after repeated chemotherapy, it has only recently been realized that, e.g., MDR1, is also found in the normal tissue such as small intestine, colon, liver and endothelial cells in the blood brain barrier. The presence of such efflux proteins in the gastro-intestinal (GI) tract, especially, in the small intestine and colon, may contribute to the poor bioavailability of many natural product drugs (including the anticancer agents vinblastine and doxorubicin). For example, many chemotherapeutic agents given orally can not show anti-tumor activity due to poor bioavailability and their inability to enter GI tissues. Furthermore, efflux proteins present in hepatocytes may additionally reduce the bioavailability of therapeutic agents by elimination via bile (see Faber et al., Adv. Drug Del. Rev., 55, 107-124, 2003).

Orally administered therapeutic agents must overcome several barriers before reaching their target site. The first major obstacle to cross is the intestinal epithelium. Although lipophilic compounds may readily diffuse across the apical plasma membrane, their subsequent passage across the basolateral membrane and into portal blood is by no means guaranteed. Efflux pump proteins located at the apical membrane, which include various drug transporters of the ATP-binding cassette (ABC) family, e.g., ABC transporters such as MDR1, MRP1 and MRP2, may drive compounds from inside the cell back into the intestinal lumen, restricting their oral bioavailability by preventing their absorption into blood. The second major hurdle to face is the liver where drugs are transported passively or by saturable transport processes from the portal blood across hepatocyte plasma (sinusoidal) membranes and bile (canalicular) membranes into bile. Efflux pump proteins located at the canalicular membranes, which again include various drug transporters of the ABC family, e.g., ABC transporters such as MDR1, breast cancer resistance protein (BCRP) and MRP2, may drive drug compounds from inside hepatocytes into the bile, restricting their oral bioavailability by promoting biliary elimination. For example, MDR1 has been demonstrated to transport most HIV protease inhibitors and to reduce their oral bioavailability and lymphocyte, brain, testis and fetal penetration, possibly resulting in major limiting effects on the therapeutic efficacy of these drugs.

Therefore, one approach to improve bioavailability may be to co-administer an efflux protein inhibitor, i.e., a compound that inhibits the function of efflux proteins, with a drug substance. In other words, when an efflux protein inhibitor is co-administered with a therapeutic agent which is also a substrate for that specific efflux system, the oral bioavailability and/or the pharmacological active concentrations at the target site of the therapeutic agent may be enhanced by inhibiting the efflux mechanism from inside the cell back into the intestinal lumen and/or by inhibiting secretion into bile.

However, efflux proteins exhibit low substrate specificity, and transport many kinds of molecules. The specificity is not rigorously understood, and there is no way of predicting from the molecular structure of a drug substance whether that specific drug will be a substrate for a certain transporter protein. Thus, it is generally not possible to predict whether a particular drug or compound will be subject to the efflux pumping action discussed above. Also, if a particular drug has a low oral bioavailability, it is generally not possible to predict whether the low bioavailability is caused, wholly or partially, by the efflux proteins discussed above, nor can it be predicted whether the low bioavailability can be increased by co-administration of an efflux protein inhibitor (see Chan et al. Eur. J. Pharmaceut. Sci., 21, 25-51, 2004).

In WO 2006/013094 discloses the use of a renin inhibitor, such as aliskiren, together with an efflux protein inhibitor, namely, the MDR1 inhibitor PSC833.

Despite the advantages conferred by this finding, there is a continuing need for improved and simple systems for enhancing the bioavailability of renin inhibitors.

Surprisingly, it has now been found that the bioavailability of many renin inhibitors, e.g., those disclosed in U.S. Patents No. 5,559,111, No. 6,197,959 and No. 6,376,672, can be significantly improved and the inter- and intra-subject variability can be decreased by using not only pharmacologically active substances such as those described in WO 2006/013094, but also non-pharmacological active compounds, including GRAS compounds and excipients, that are MDR1 inhibitors in combination with renin inhibitors.

Thus, the present invention relates to a pharmaceutical composition comprising

(i) a renin inhibitor and

(ii) an MDR1 inhibitor selected from a non-pharmacologically active compound.

Accordingly, the present invention provides also a method for improving the bioavailability, preferably, oral bioavailability, of a renin inhibitor, which method comprises co-administering to a mammal, especially a human, in need of such treatment, a combination of a renin inhibitor and an MDR1 inhibitor selected from non-pharmacologically active compounds including GRAS compounds and excipients, in particular a GRAS compound. The non-pharmacologically active compound is administered in an amount such that the bioavailability of a renin inhibitor is improved in comparison with what the bioavailability would be in the absence of the an MDR1 inhibitor selected from a non-pharmacologically active compound (e.g. 10 % when administered orally to humans). An MDR1 inhibitor selected from a non-pharmacologically active compound and a renin inhibitor are preferably each co-administered in an amount such that the combination has a desired therapeutic effect, e.g., an antihypertensive effect.

In particular, the present invention provides a method for improving the bioavailability of a δ-amino-γ-hydroxy-ω-aryl-alkanoic acid derivative, which method comprises co-administering to a mammal, especially a human, in need of such treatment, a combination of a δ-amino-γ-hydroxy-ω-aryl-alkanoic acid derivative, or a pharmaceutically acceptable salt thereof, and an MDR1 inhibitor selected from a non-pharmacologically active compound.

Figure 1 shows the effect of the renin inhibitor Aliskiren on the ATPase activity in membrane vesicles expressing high levels of MDR1.

Figure 2 shows bi-directional transport of the renin inhibitor Aliskiren across Caco-2 cell monolayers in the apical (AP) to basolateral (BL) and BL-to-AP direction.

The term "co-administration" of a combination of a renin inhibitor, in particular, a δ-amino-γ-hydroxy-ω-aryl-alkanoic acid derivative, and an MDR1 inhibitor selected from a non-pharmacologically active compound means that the two components can be administered together as a pharmaceutical composition or as part of the same, unitary dosage form. Co-administration also includes administering a renin inhibitor, in particular, a δ-amino-γ-hydroxy-ω-aryl-alkanoic acid derivative, and an MDR1 inhibitor selected from a non-pharmacologically active compound separately but as part of the same therapeutic regimen. The two components, if administered separately, need not necessarily be administered at essentially the same time, although they can if so desired. Thus, co-administration includes, for example, administering a renin inhibitor, in particular, a δ-amino-γ-hydroxy-ω-aryl-alkanoic acid derivative, plus a an MDR1 inhibitor selected from a non-pharmacologically active compound as separate dosages or dosage forms, but at the same time. Co-administration also includes separate administration at different times and in any order.

The renin inhibitors to which the present invention applies are any of those having renin inhibitory activity *in vivo* and, therefore, pharmaceutical utility, e.g., as therapeutic agents for the treatment of hypertension, congestive heart failure, cardiac hypertrophy, cardiac fibrosis, cardiomyopathy postinfarction, complications resulting from diabetes, such as nephropathy, vasculopathy and neuropathy, diseases of the coronary vessels, restenosis following angioplasty, raised intra-ocular pressure, glaucoma, abnormal vascular growth, hyperaldosteronism, anxiety states and cognitive disorders. In particular, the present invention relates to δ-amino-γ-hydroxy-ω-aryl-alkanoic acid amide derivatives as disclosed in U.S. Patent No. 5,559,111.

A renin inhibitor, in particular, a δ-amino-γ-hydroxy-ω-aryl-alkanoic acid derivative, of the present invention may be employed in the form of its pharmaceutically acceptable salts, in an anhydrous form or a hydrate or a solvate thereof. All such forms are useful within the scope of the present invention.

Preferably, a δ-amino-γ-hydroxy-ω-aryl-alkanoic acid derivative of the present invention having the formula

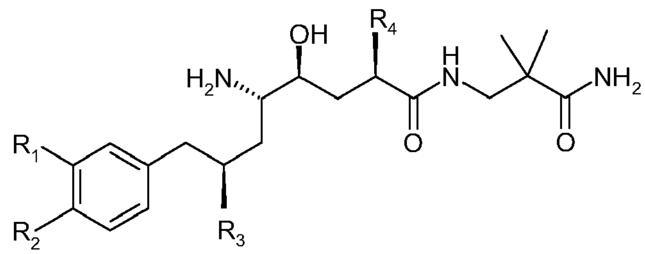

(I)

wherein $R_1$ is $C_{1-4}$alkoxy-$C_{1-4}$alkoxy or $C_{1-4}$alkoxy-$C_{1-4}$alkyl; $R_2$ is $C_{1-4}$alkyl or $C_{1-4}$alkoxy; and $R_3$ and $R_4$ are independently branched $C_{1-4}$alkyl; or a pharmaceutically acceptable salt thereof; is used.

More preferably, a δ-amino-γ-hydroxy-ω-aryl-alkanoic acid derivative of the present invention having formula (I) wherein $R_1$ is 3-methoxypropoxy; $R_2$ is methoxy; and $R_3$ and $R_4$ are isopropyl; or a pharmaceutically acceptable salt thereof; most preferably, a δ-amino-γ-hydroxy-ω-aryl-alkanoic acid derivative of the present invention which is (2S,4S,5S,7S)-5-amino-4-hydroxy-2-isopropyl-7-[4-methoxy-3-(3-methoxy-propoxy)-benzyl]-8-methyl-nonanoic acid (2-carbamoyl-2-methyl-propyl)-amide hemifumarate, also known as Aliskiren, is employed.

The term "MDR1 inhibitor" as used herein is intended to encompass efflux protein inhibitors, namely inhibitors

of multidrug resistance protein type transporters. The term "efflux protein inhibitor" as used herein refers to any compound, a pharmaceutical or an excipient compound, that inhibits the action of a ABC transporter, e.g. those disclosed in Bakos et al. Mol Pharmacol., 57, 760-768 (2002) and Maarten et al. AIDS, 16, 2295-2301 (2002). Additional information on efflux protein inhibitors can be found in WO 2006/013094.

The term "non-pharmacologically active compound" as used herein is defined according to the EMEA guidelines as a compound which is not pharmacodynamically active at the dose at which it is administered to the target, such as the mammal, including the human, by means of a medicinal product in which it is included, but it may be capable of pharmacological action when incorporated at a different concentration in another product.

Thus, the MDR1 inhibitor selected from a non-pharmacologically active compound possess, when employed in accordance with the present invention, no other pharmacological action than the MDR1 inhibition.

The term "GRAS compound" as used herein is a commonly employed term used by the health authorities, in particular the FDA, and is the acronym for Generally Recognized As Safe. It is a specifically-designated regulatory category for conventional foods. Ingredients added to conventional foods must be approved as food additives or be GRAS, both of which assure "reasonable certainty" of safety. In general, GRAS compounds are non-toxic and themselves not pharmacologically active. In this context, the term "non-pharmacologically active" means that the GRAS compounds themselves, at least in the amount and concentration in which they are employed, do not to cure or reduce symptoms of an illness or medical conditions.

Ingredients commonly used in food prior to 1958, when the Food Additives Amendment was added to the Federal Food, Drug and Cosmetic Act (FFDCA), are automatically GRAS, as are substances for which relative scientific agreement exists as to the safety.

In 1997, a GRAS self-affirmation notification process was proposed; while it has not been finalized, FDA is operating under the aegis of the rule with industry buy-in. The GRAS process is now essentially an independent process with the same safety requirements as food additives, but without FDA approval. In the process of self-affirmation, companies work to assemble a comprehensive dossier of scientific substantiation and historical documentation supporting the safe use of the compound. Because of the requirement of "general recognition", the pivotal data for safety substantiation must be part of the public domain. During the review process, companies work to define particular food categories of interest as well as the usage levels for those categories. The final determination by an expert panel includes reviewing technical evidence about the safety of the ingredient for its intended use and common knowledge about the safety of the ingredient, with the data providing "reasonable certainty" of safety. The health authorities regularly publish lists of GRAS compounds.

The term "excipient" as used herein is defined according to the EMEA guidelines as a constituent of a pharmaceutical form or medicinal product, other than the active substance. Thus, an excipient is an inactive constituent used as a carrier for the active substance of the medicinal product. Excipients can be used to aid the manufacturing process of the medicinal product, ease administration of the medicinal product by bringing it into an appropriate form or to bulk up formulations to allow for convenient and accurate dosage.

The non-pharmacologically active compounds suitable for the pharmaceutical composition in accordance with the present invention can be any non-pharmacologically active compound which is an MDR1 inhibitor. Preferably the non-pharmacologically active compounds include GRAS compounds and excipients which are MDR1 inhibitors.

Typically, non-limiting examples of GRAS compounds that are MDR1 inhibitors include:

Curcumin

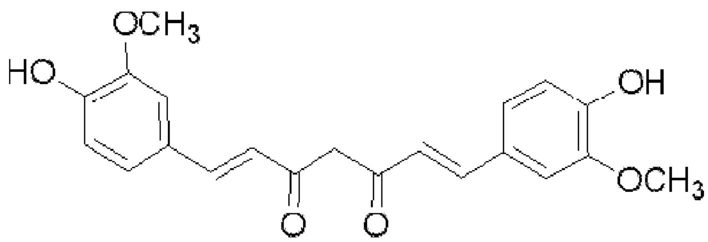

Phenyl cinnamate

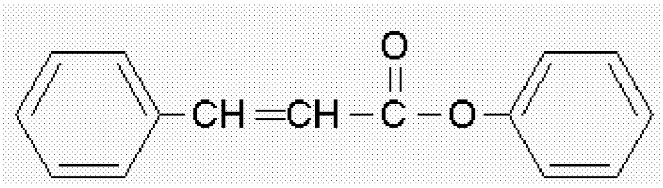

Coumarin

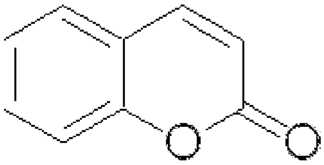

Beta-amyrin cinnamate

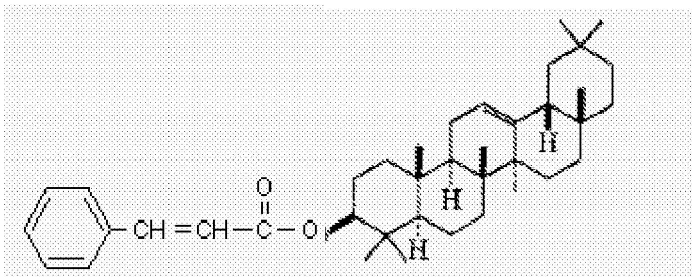

Apiole
Bergamottin

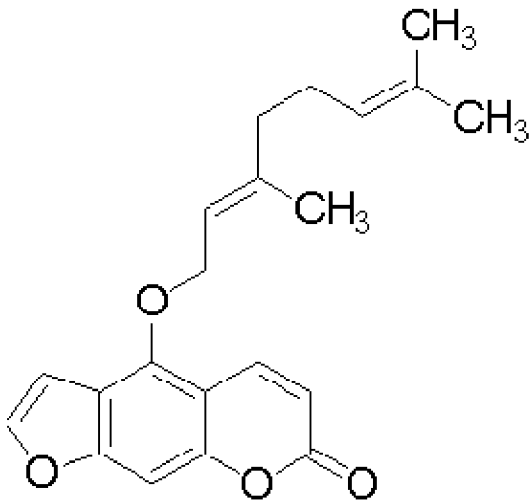

Caffein

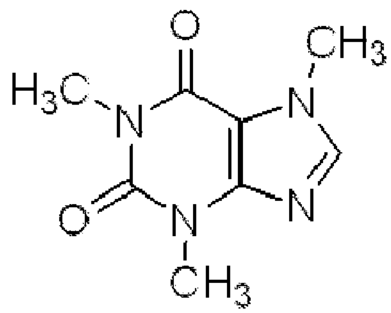

Caffeine, 8-(Decylthio-)
Caffeine, 8-benzyl
Diethylpyrocarbonate
Morin

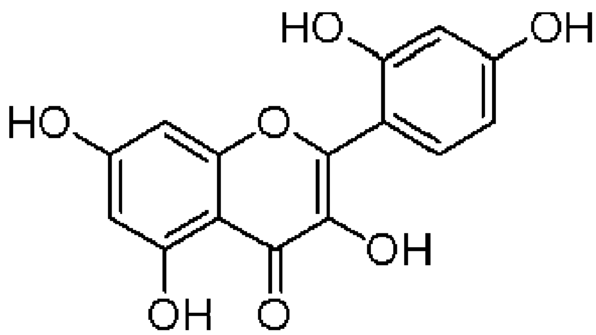

Narirutin
Piperine

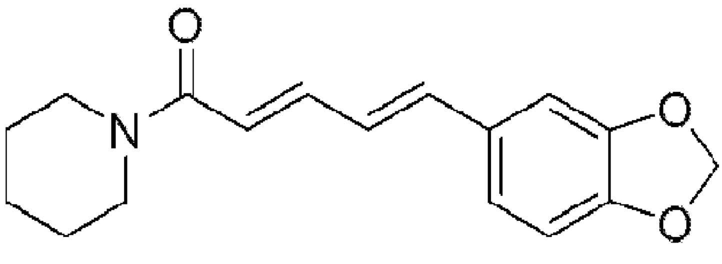

Quercetin

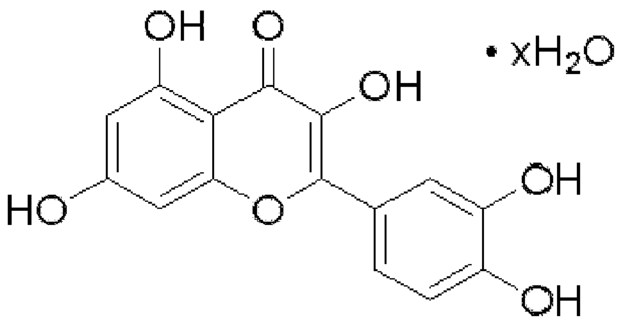

Slavironin
Silybin
Theobromin

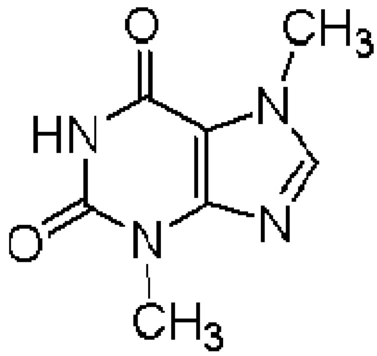

Vanillin

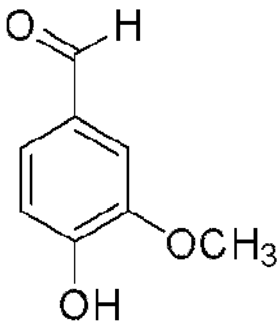

and Vanillyl-N-nonlymine.

Typically, non-limiting examples of excipients which are MDR1 inhibitors include surfactants, in particular non-ionic surfactants, as listed below:

1) Reaction products of a natural or hydrogenated castor oil and ethylene oxide

The natural or hydrogenated castor oil may be reacted with ethylene oxide in a molar ratio of from about 1:35 to about 1:60, with optional removal of the polyethylene-glycol component from the products. Various such surfactants are commercially available. Particularly suitable surfactants include polyethyleneglycol-hydrogenated castor oils available under the trade name Cremophor®; Cremophor® RH 40, which has a saponification value of about 50 to 60, an acid value less than about 1, a water content (Fischer) less than about 2%, an $n_D^{60}$ of about 1.453- to 1.457 and an HLB of about 14 14-to 16; and Cremophor® RH 60, which has a saponification value of about 40- to 50, an acid value less than about 1, an iodine value of less than about 1, a water content (Fischer) of about 4.5- to 5.5%, an $n_D^{60}$ of about 1.453 453-to 1.457 and an HLB of about 15 to 17.

An especially preferred product of this class is Cremophor® RH40. Other useful products of this class are available under the trade names Nikkol® (e.g. Nikkol® HCO-40 and HCO-60), Mapeg® (e.g. Mapeg® CO-40h), Incrocas® (e.g. Incrocas® 40), Tagat® (for example polyoxyethylene-glycerol-fatty acid esters e.g. Tagat® RH 40) and Simulsol OL-50 (PEG-40 castor oil, which has a saponification value of about 55 to 65, an acid value of max. 2, an iodine value of 25 to 35, a water content of max. 8%, and an HLB of about 13, available from Seppic). These surfactants are further described in Fiedler *loc. cit.*loc. cit..

Other suitable surfactants of this class include polyethyleneglycol castor oils such as that available under the trade name Cremophor® EL, which has a molecular weight (by steam osmometry) of about 1630, a saponification value of about 65 to 70, an acid value of about 2, an iodine value of about 28 to 32 and an $n_D^{25}$ of about 1.471.

2) Polyoxyethylene-sorbitan-fatty acid esters

These include , for example mono- and tri-lauryl, palmityl, stearyl and oleyl esters of the type known and commercially available under the trade name Tween® (Fiedler, *loc. cit.* loc. cit. p.1615 ff) from Uniqema including the products:

Tween@ 20 [polyoxyethylene(20)sorbitanmonolaurate],
Tween@ 21 [polyoxyethylene(4)sorbitanmonolaurate],
Tween@ 40 [polyoxyethylene(20)sorbitanmonopalmitate],
Tween@ 60 [polyoxyethylene(20)sorbitanmonostearate],
Tween@ 65 [polyoxyethylene(20)sorbitantristearate],
Tween@ 80 [polyoxyethylene(20)sorbitanmonooleate],
Tween@ 81 [polyoxyethylene(5)sorbitanmonooleate], and
Tween@ 85 [polyoxyethylene(20)sorbitantrioleate].

Especially preferred products of this class are Tween@ 20 and Tween@ 80.

3) Polyoxyethylene-polyoxypropylene co-polymers and block co-polymers or, poloxamers
These include , for example of the type known and commercially available under the trade names Pluronic® and Emkalyx® (Fiedler, *loc. cit.*loc. cit., 2, p. 1203). An especially preferred product of this class is Pluronic® F68 (poloxamer 188) from BASF, having a melting point of about 52°C and a molecular weight of about 6800 to 8975.

4) Polyoxyethylene mono esters of a saturated $C_{10}$ to $C_{22}$,
These include e.g. $C_{18}$ substituted e.g. hydroxy fatty acid; e.g. 12 hydroxy stearic acid PEG ester, e.g. of PEG about e.g. 600-900 e.g. 660 Daltons MW, e.g. Solutol® HS 15 from BASF, Ludwigshafen, Germany.
According to the BASF technical leaflet MEF 151E (1986) comprises about 70% polyethoxylated 12-hydroxystearate by weight and about 30% by weight unesterified polyethylene glycol component. Solutol HS 15 has a hydrogenation value of 90 to 110, a saponification value of 53 to 63, an acid number of maximum 1, and a maximum water content of 0.5% by weight.

5) Water soluble tocopheryl polyethylene glycol succinic acid esters (TPGS)
These include , e.g. those with a polymerisation number ca 1000, e.g. available from Eastman Fine Chemicals Kingsport, Texas, USA.

6) Transesterified, polyoxyethylated caprylic-capric acid glycerides
These include for example those that are commercially available under the trade name Labrasol® from e.g. Gattefossé. Labrasol® has an acid value of max. 1, a saponification value of 90-110, and an iodine value of max. 1 (H. Fiedler, *loc. cit.*loc. cit., vol 2, page 880).

Of these non-ionic surfactants, the following are particularly preferred: Vitamin E TPGS, Cremophor EL, Cremophor RH40, Polysorbat 80, Solutol HS15, Pluronic F68, Labrasol.

Of these non-pharmacologically active compounds, the following are particularly preferred: Curcumin, Vitamin E TPGS, Piperine, Coumarin, and Phenyl cinnamate.

It was surprisingly found that these compounds, when co-administered with a renin inhibitor, such as aliskiren, could significantly increase the bioavailability of this compound and the inter- and intra-subject variability decreased. Due to the increase of bioavailability the amount of drug substance can be significantly decreased and, thus, the costs per medicament. In addition, since these MDR1 inhibitors are by definition non-toxic and non-pharmacologically active, there is no need to perform extensive studies for approval by the health authorities. To the contrary, these non-pharmacologically active compounds will be considered just as excipients and this will further decrease the development time and costs.

As disclosed herein above, a renin inhibitor, in particular, a δ-amino-γ-hydroxy-ω-aryl-alkanoic acid derivative, and an MDR1 inhibitor selected from a non-pharmacologically active compound can be co-administered as a pharmaceutical composition. The components may be administered together in any conventional dosage form, usually also together with a pharmaceutically acceptable carrier or diluent.

For oral administration the pharmaceutical composition comprising a renin inhibitor, in particular, a δ-amino-γ-hydroxy-ω-aryl-alkanoic acid derivative, and an MDR1 inhibitor selected from a non-pharmacologically active compound can take the form of solutions, suspensions, tablets, pills, capsules, powders, microemulsions, unit dose packets and the like. Preferred are tablets and gelatin capsules comprising the active ingredient together with: a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrrolidone; if desired

d) disintegrants, e.g., starches, agar, alginic acid or its sodium salt, or effervescent mixtures; and/or e) absorbants, colorants, flavors and sweeteners. Injectable compositions are preferably aqueous isotonic solutions or suspensions, and suppositories are advantageously prepared from fatty emulsions or suspensions.

Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, preferably about 1-50%, of the active ingredient.

More specifically, the present invention provides a pharmaceutical composition comprising a therapeutically effective amount of a renin inhibitor, preferably, a δ-amino-γ-hydroxy-ω-aryl-alkanoic acid derivative, in combination with an MDR1 inhibitor selected from a non-pharmacologically active compound, said an MDR1 inhibitor selected from a non-pharmacologically active compound being preferably present in an amount such that, following administration, the bioavailability of a renin inhibitor is improved by at least 5 %.

Preferably, a pharmaceutical composition of the present invention comprises a δ-amino-γ-hydroxy-ω-aryl-alkanoic acid derivative of the formula

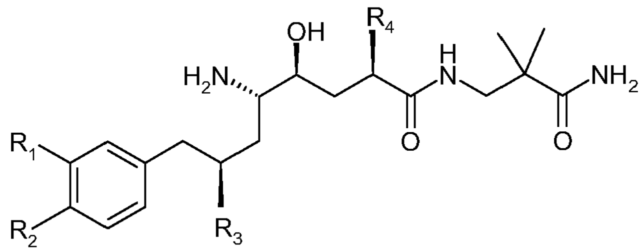

(I)

wherein $R_1$ is $C_{1-4}$alkoxy-$C_{1-4}$alkoxy or $C_{1-4}$alkoxy-$C_{1-4}$alkyl; $R_2$ is $C_{1-4}$alkyl or $C_{1-4}$alkoxy; and $R_3$ and $R_4$ are independently branched $C_{1-4}$alkyl; or a pharmaceutically acceptable salt thereof; in combination with a an MDR1 inhibitor selected from a non-pharmacologically active compound.

More preferably, a pharmaceutical composition of the present invention comprises a δ-amino-γ-hydroxy-ω-aryl-alkanoic acid derivative of formula (I) wherein $R_1$ is 3-methoxypropoxy; $R_2$ is methoxy; and $R_3$ and $R_4$ are isopropyl; or a pharmaceutically acceptable salt thereof; in combination with an MDR1 inhibitor selected from a non-pharmacologically active compound.

Most preferably, a pharmaceutical composition of the present invention comprises (2S,4S,5S,7S)-5-amino-4-hydroxy-2-isopropyl-7-[4-methoxy-3-(3-methoxy-propoxy)-benzyl]-8-methyl-nonanoic acid (2-carbamoyl-2-methyl-propyl)-amide hemifumarate in combination with an MDR1 inhibitor selected from a non-pharmacologically active compound.

Preferably, the bioavailability of a renin inhibitor, in particular, a δ-amino-γ-hydroxy-ω-aryl-alkanoic acid derivative, e.g., Aliskiren, or a pharmaceutically acceptable salt thereof, is improved by at least 5 %.

Bioavailability of a drug may be assessed as known in the art by measuring AUCs, where AUC is the area under the curve (AUC) plotting the serum or plasma concentration of a drug along the ordinate (Y-axis) against time along the abscissa (X-axis). Generally, the values for AUC represent a number of values taken from all the subjects in a test population and are, therefore, mean values averaged over the entire test population.

Co-administration a renin inhibitor and an MDR1 inhibitor selected from a non-pharmacologically active compound may also increase $C_{max}$ relative to dosing the renin inhibitor in the absence of an efflux protein inhibitor, and this is provided as a further aspect of the invention. $C_{max}$ is also well understood in the art as an abbreviation for the maximum drug concentration in serum or plasma of a test subject.

Since the present invention has an aspect that relates to treatment with a combination of compounds which may be co-administered separately, the invention also relates to combining separate pharmaceutical compositions in kit form. The kit comprises two separate pharmaceutical compositions: (1) a composition comprising a renin inhibitor, in particular, a δ-amino-γ-hydroxy-ω-aryl-alkanoic acid derivative, plus a pharmaceutically acceptable carrier or diluent; and (2) a composition comprising an MDR1 inhibitor selected from a non-pharmacologically active compound plus a pharmaceutically acceptable carrier or diluent. The amounts of (1) and (2) are such that, when co-administered separately, the bioavailability of a renin inhibitor, in particular, a δ-amino-γ-hydroxy-ω-aryl-alkanoic acid derivative, is improved preferably by at least 5 %. The kit comprises a container for containing the separate compositions such as a divided bottle or a divided foil packet, wherein each compartment contains a plurality of dosage forms (e.g., tablets) comprising (1) or (2). Alternatively, rather than separating the active ingredient-containing dosage forms, the kit may contain separate compartments each of which contains a whole dosage which in turn comprises separate dosage forms. An example of this type of kit is a blister pack wherein each individual blister contains two (or more) tablets, one (or more) tablet(s) comprising a pharmaceutical composition (1), and the second (or more) tablet(s) comprising a pharmaceutical composition (2). Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician. In the case of the instant invention a kit therefore comprises:

(1) a therapeutically effective amount of a composition comprising a renin inhibitor, in particular, a δ-amino-γ-hydroxy-ω-aryl-alkanoic acid derivative, e.g., Aliskiren, or a pharmaceutically acceptable salt thereof, and a pharmaceutically acceptable carrier or diluent, in a first dosage form;

(2) a composition comprising an MDR1 inhibitor selected from a non-pharmacologically active compound in an amount such that, following administration, the bioavailability of a renin inhibitor, in particular, a δ-amino-γ-hydroxy-ω-aryl-alkanoic acid derivative, e.g., aliskiren, or a pharmaceutically acceptable salt thereof, is preferably improved by at least 5 %, and a pharmaceutically acceptable carrier or diluent, in a second dosage form; and

(3) a container for containing said first and second dosage forms.

Ultimately, the present invention relates to a use of a an MDR1 inhibitor selected from a non-pharmacologically active compound, for the manufacture of a medicament to improve the bioavailability, preferably oral bioavailability, of a renin inhibitor, preferably, a δ-amino-γ-hydroxy-ω-aryl-alkanoic acid derivative, e.g., Aliskiren, or a pharmaceutically acceptable salt thereof.

The efflux protein(s) involved in the drug efflux of a drug substance may be identified, and the corresponding kinetic parameters may be determined, i.e., Michaelis-Menten Constant and Maximal Drug Transport ($K_m$ and $J_{max}$), using methods known in the art, e.g., by an ATPase assay using Sf9 (Spodoptera fruigiperda) membrane vesicles expressing high levels of the selected ABC transporter. In this assay the ABC transporters remove substrates out of cells by using ATP hydrolysis as an energy source. ATP hydrolysis yields inorganic phosphate (Pi), which can be detected by a simple colorimetric reaction. The amount of Pi liberated by the transporter is proportional to the activity of the transporter. Membrane preparations containing ABC transporters show a baseline ATPase activity that varies for different transporters. Transported substrates increase this baseline ATPase activity, while inhibitors inhibit the baseline ATPase activity and/or the ATPase activity measured in the presence of a stimulating agent. Both, activation and inhibition studies may be performed. As illustrated herein in Example 1 (Figure 1), Aliskiren increases the ATPase activity in membrane vesicles expressing high levels of MDR1 with a $K_m$ value of about 3 μM, suggesting that the efflux system involved in Aliskiren transport is possibly MDR1.

Alternatively, the *in vitro* transporter affinity of a drug substance can be determined and approximated by a Caco-2 cell assay as described, e.g., in Camenisch et al., Pharm. Act. Helv. 71, 309-327 (1996), or as illustrated herein in the Examples. The identification of the transporter protein and the efficacy of a compound to inhibit the efflux system involved may as well be determined in the Caco-2 cell assay. For example, Aliskiren is identified as a low to moderate permeable compound (intrinsic permeability < 80%), being additionally a substrate for a prominent efflux system (Figure 2).

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore, the Examples herein are to be construed as merely illustrative and not a limitation of the scope of the present invention in any way.

## Example 1:

### ATPase assay

Efflux mediated by the human MDR1, is measured by incubating the purified membrane vesicles in the absence and the presence of a stimulating agent (Verapamil [40 μM] for MDR1) with different concentrations of a drug substance [0.046, 0.137, 0.41, 1.23, 3.7, 11.1, 33.3 and 100 μM] in transport buffer at pH 7.4 at 37°C.

A 5 mM stock solution of the therapeutic agent of interest will be prepared in a common organic solvent, e.g., dimetylsulfoxide, ethanol, methanol and acetonitrile, such a way that addition of the stock solution or its dilutions into assay mixture produces the above mentioned final concentrations, and the organic solvent used is 2 % of the total volume (v/v). All the solutions used in this assay will be maintained at pH 7.4.

Membrane vesicles maintained at -80°C will be used for the ATPase experiments. Transporter mediated efflux may be determined as described in literature (Sarkadi, B. Price, E. Boucher, R. Germann, U. and Scarborough, G. J. Biol. Chem. 1992, 267: 4854-4858). Briefly, membrane suspension in the presence and absence of a test drug, stimulating

agent, $Na_3VO_4$ 60 mM and Glutathione 2 mM (only for MRP1 and MRP2 transporters) is pipetted into a 96-well plate and transferred to 37°C for 5 min of preincubation. The ATPase reaction is started by addition of 25 mM Mg-ATP solution and subsequent incubation at 37°C (20 min for MDR1, 60 min for MRP1 and 30 min for MRP2). Afterwards, the ATPase reaction is stopped by adding SDS (5%) to each incubation. After addition of ammonium molybdate/zinc acetate colorimetric detection reagent the plate is incubated for an addition 25 min at 37°C.

After incubating the OD is measured at 730 nm. Using a previously determined phosphate standard curve the Pi liberated [nmol/well] can be calculated. OD values will be presented as means ± standard deviations of the experiments performed (n = 2). All statistical analyses are performed using Microsoft EXCEL 5.0c.

To calculate the so-called specific ($Na_3VO_4$ sensitive) transporter ATPase activity for each drug and drug-concentration assayed the Pi values determined in the presence of $Na_3VO_4$ have to be subtracted from the Pi values measured without $Na_3VO_4$. The $Na_3VO_4$ sensitive transporter activity in terms of Pi liberated/mg membrane protein/min can be determined by dividing the numbers by the amount of membrane protein added to each well and the time of incubation in min (Figure 1).

**Example 2:**

**Caco-2 cell assay**

Caco-2 cell monolayers grown on PET filters for 21-25 days are used for the transport experiments. The flux of compounds across Caco-2 cell monolayers grown on PET filters as well as across PET filters alone without Caco-2 cells is determined as follows: Prior to the transport experiment, the culture medium in the acceptor compartment (0.2 mL for apical and 1.0 mL for basolateral sides) is replaced with acceptor solution (HBSS, when relevant containing the inhibitor of interest) preincubated at 37°C. To start the experiment, the medium in the donor compartment (0.35 mL for apical and 1.15 mL for basolateral sides) is replaced with donor solution (compound in HBSS, when relevant containing inhibitor of interest) pre-incubated at 37°C. Aliquots of 150 μL are removed from the donor and the acceptor side after about 1 and 120 min. Transport experiments in both apical-to-basolateral and basolateral-to-apical directions are performed in triplicate at 37°C in an incubator without shaking.

The suitability of Caco-2 cells for transport experiments is examined by measuring the permeability of [$^3$H]-mannitol at ≤ 0.1 μM and [$^3$H]-propranolol at ≤ 0.1 μM from apical to basolateral sides for 120 min in a total of 6 representative cell monolayers (3 for each compound) within the same batch of cells.

Radioactive samples are analyzed by liquid scintillation counting. All other non-radiolabeled samples are kept frozen at -20°C until analysis by liquid chromatography/tandem mass spectrometry (LC-MS/MS).

Transport values of the compounds tested are determined using the following equation (Artursson et al., Biochem. Biophys. Res. Comm. 175: 880-885, 1991):

$$P_{app} = \frac{\Delta Q}{\Delta t \cdot A \cdot C_0}$$

where $P_{app}$ (cm/min) is the apparent permeability coefficient, ΔQ is the amount of compound found in the acceptor compartment at time t, Δt (min) is the incubation time period, $C_0$ (μg/mL) is the initial concentration of the compound in the donor compartment and A (cm$^2$) is the surface area of the membrane.

For labeled samples, the limit of quantitation (LOQ) is taken as the lowest sample concentration value obtained from the radioactive scale which is significantly higher than the measured blank value and for which the standard error of the measurement is lower than 20 %. Under the conditions of this study, the LOQ of absolute radioactivity is 2 dpm for [$^{14}$C]-labeled Aliskiren corresponding to 12 nmol/L.

$P_{app}$ values are presented as means ± standard deviations of the transport experiments performed (n = 3). The statistical significance in differences between any two given data sets is examined by *t*-test. The probability level for assignment of significance of difference is p < 0.025. All statistical analyses were performed using Microsoft EXCEL 5.0c.

For 1 μM concentration of Aliskiren within a time period of 120 min an apical to basolateral transport of about $0.2 \cdot 10^{-5}$ cm/min is detectable. Basolateral to apical transport on the other side occurred with a permeability value of about $10 \cdot 10^{-5}$ cm/min, which is significantly higher than the apical to basolateral transport.

For 1, 5, 10 and 50 μM concentration of Aliskiren a gradual increase of apical to basolateral transport is observed, approaching a plateau permeability value of about $7 \cdot 10^{-5}$ cm/min at 10 μM. Basolateral to apical transport on the other side does not change significantly with increasing concentrations.

The recovery values for Caco-2 transport of Aliskiren (1, 5, 10 and 50 μM) are generally very high (< 100 %),

indicating that Aliskiren does not bind to the filter support or the plastic incubation environment.

Apical to basolateral flux for the paracellular marker Mannitol and the transcellular marker Propranolol are always below the threshold $P_{app}$ values of $3 \cdot 10^{-5}$ cm/min and $90 \cdot 10^{-5}$ cm/min, respectively. The determined apical to basolateral filter permeabilities are generally higher than the corresponding Caco-2 permeability data, indicating filter diffusion not to be the rate limiting step for Caco-2 transport (Figures 2).

## Claims

1. A pharmaceutical composition comprising

   (i) a renin inhibitor and
   (ii) an MDR1 inhibitor selected from a non-pharmacologically active compound.

2. The pharmaceutical composition according to claim 1, wherein the non-pharmacologically active compound is a GRAS compound or an excipient.

3. The pharmaceutical composition according to claim 1, wherein the non-pharmacologically active compound is a GRAS compound selected from the group consisting of curcumin, phenyl cinnamate, coumarin, beta-amyrin cinnamate, apiole, bergamottin, caffein, 8-(decylthio-)caffeine, 8-benzyl-caffeine, diethylpyrocarbonate, morin, narirutin, piperine, quercetin, slavironin, silybin, theobromin, vanillin, and vanillyl-N-nonlymine, or is an excipient selected from non-ionic surfactants, including vitamin E TPGS and Cremophor EL.

4. The pharmaceutical composition according to any of claims 1 to 3 wherein the non-pharmacologically active compound is Curcumin, Vitamin E TPGS, Piperine, Coumarin, and Phenyl cinnamate

5. The pharmaceutical composition according to any of the preceding claims, wherein the renin inhibitor is a δ-amino-γ-hydroxy-ω-aryl-alkanoic acid amide derivative, or a pharmaceutically acceptable salt thereof.

6. The pharmaceutical composition according to claim 4, wherein the δ-amino-γ-hydroxy-ω-aryl-alkanoic acid amide derivative has the formula

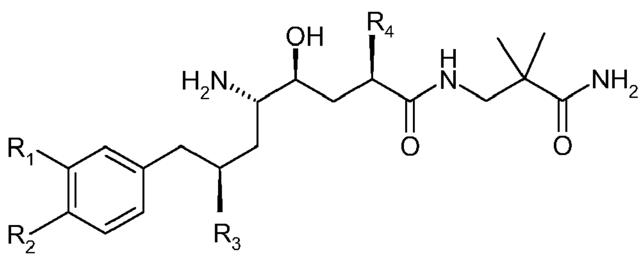

(I)

   wherein $R_1$ is $C_{1-4}$alkoxy-$C_{1-4}$alkoxy or $C_{1-4}$alkoxy-$C_{1-4}$alkyl; $R_2$ is $C_{1-4}$alkyl or $C_{1-4}$alkoxy; and $R_3$ and $R_4$ are independently branched $C_{1-4}$alkyl; or a pharmaceutically acceptable salt thereof.

7. The pharmaceutical composition according to claim 5 or 6, wherein the δ-amino-γ-hydroxy-ω-aryl-alkanoic acid amide derivative is a compound of formula (I) wherein $R_1$ is 3-methoxypropoxy; $R_2$ is methoxy; and $R_3$ and $R_4$ are isopropyl; or a pharmaceutically acceptable salt thereof.

8. The pharmaceutical composition according to Claim 7, wherein the δ-amino-γ-hydroxy-ω-aryl-alkanoic acid amide derivative is (2S,4S,5S,7S)-5-amino-4-hydroxy-2-isopropyl-7-[4-methoxy-3-(3-methoxy-propoxy)-benzyl]-8-methyl-nonanoic acid (2-carbamoyl-2-methyl-propyl)-amide hemifumarate.

9. A method of improving the bioavailability of a renin inhibitor, which method comprises co-administering, to a mammal in need such treatment, a combination of a renin inhibitor and an MDR1 inhibitor selected from a non-pharmacologically active compound.

**10.** The method according to claim 9, wherein the non-pharmacologically active compound is a GRAS compound selected from the group consisting of curcumin, phenyl cinnamate, coumarin, beta-amyrin cinnamate, apiole, bergamottin, caffein, 8-(decylthio-)caffeine, 8-benzyl-caffeine, diethylpyrocarbonate, morin, narirutin, piperine, quercetin, slavironin, silybin, theobromin, vanillin, and vanillyl-N-nonlymine, or is an excipient selected from non-ionic surfactants, including vitamin E TPGS and Cremophor EL..
The method according to claim 9 or 10 wherein the non-pharmacologically active compound is Curcumin, Vitamin E TPGS, Piperine, Coumarin, and Phenyl cinnamate.

**11.** The method according to any of claims 9 to 11, wherein the renin inhibitor is a δ-amino-γ-hydroxy-ω-aryl-alkanoic acid amide derivative, or a pharmaceutically acceptable salt thereof.

**12.** The method according to claim 12, wherein the δ-amino-γ-hydroxy-ω-aryl-alkanoic acid amide derivative has the formula

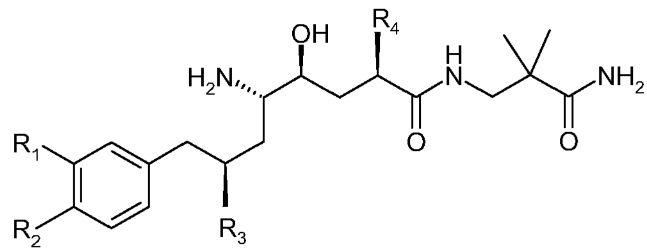

(I)

wherein $R_1$ is $C_{1-4}$alkoxy-$C_{1-4}$alkoxy or $C_{1-4}$alkoxy-$C_{1-4}$alkyl; $R_2$ is $C_{1-4}$alkyl or $C_{1-4}$alkoxy; and $R_3$ and $R_4$ are independently branched $C_{1-4}$alkyl; or a pharmaceutically acceptable salt thereof.

**13.** The method according to claim 13, wherein the δ-amino-γ-hydroxy-ω-aryl-alkanoic acid amide derivative is a compound of formula (I) wherein $R_1$ is 3-methoxypropoxy; $R_2$ is methoxy; and $R_3$ and $R_4$ are isopropyl; or a pharmaceutically acceptable salt thereof.

**14.** The method according to claim 14, wherein the δ-amino-γ-hydroxy-ω-aryl-alkanoic acid amide derivative is (2S,4S,5S,7S)-5-amino-4-hydroxy-2-isopropyl-7-[4-methoxy-3-(3-methoxy-propoxy)-benzyl]-8-methyl-nonanoic acid (2-carbamoyl-2-methyl-propyl)-amide hemifumarate.

**15.** The use of an MDR1 inhibitor selected from a non-pharmacologically active compound for the manufacture of a medicament to improve the bioavailability of a renin inhibitor, or a pharmaceutically acceptable salt thereof.

**16.** The use according to claim 15, wherein the non-pharmacologically active compound is a GRAS compound selected from the group consisting of curcumin, phenyl cinnamate, coumarin, beta-amyrin cinnamate, apiole, bergamottin, caffein, 8-(decylthio-)caffeine, 8-benzyl-caffeine, diethylpyrocarbonate, morin, narirutin, piperine, quercetin, slavironin, silybin, theobromin, vanillin, and vanillyl-N-nonlymine, or is an excipient selected from non-ionic surfactants, including vitamin E TPGS and Cremophor EL..

**17.** The use according to claim 15 or 16 wherein the non-pharmacologically active compound is Curcumin, Vitamin E TPGS, Piperine, Coumarin, and Phenyl cinnamate.

**18.** The use according to any of claims 15 to 17, wherein the renin inhibitor is a δ-amino-γ-hydroxy-ω-aryl-alkanoic acid amide derivative, or a pharmaceutically acceptable salt thereof.

**19.** The use according to claim 18, wherein the δ-amino-γ-hydroxy-ω-aryl-alkanoic acid amide derivative has the formula

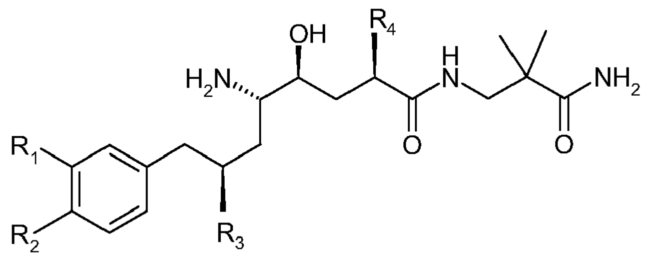

(I)

wherein $R_1$ is $C_{1-4}$alkoxy-$C_{1-4}$alkoxy or $C_{1-4}$alkoxy-$C_{1-4}$alkyl; $R_2$ is $C_{1-4}$alkyl or $C_{1-4}$alkoxy; and $R_3$ and $R_4$ are independently branched $C_{1-4}$alkyl; or a pharmaceutically acceptable salt thereof.

**20.** The use according to claim 19, wherein the δ-amino-γ-hydroxy-ω-aryl-alkanoic acid amide derivative is a compound of formula (I) wherein $R_1$ is 3-methoxypropoxy; $R_2$ is methoxy; and $R_3$ and $R_4$ are isopropyl; or a pharmaceutically acceptable salt thereof.

**21.** The use according to claim 20, wherein the δ-amino-γ-hydroxy-ω-aryl-alkanoic acid amide derivative is (2S,4S,5S, 7S)-5-amino-4-hydroxy-2-isopropyl-7-[4-methoxy-3-(3-methoxy-propoxy)-benzyl]-8-methyl-nonanoic acid (2-carbamoyl-2-methyl-propyl)-amide hemifumarate.

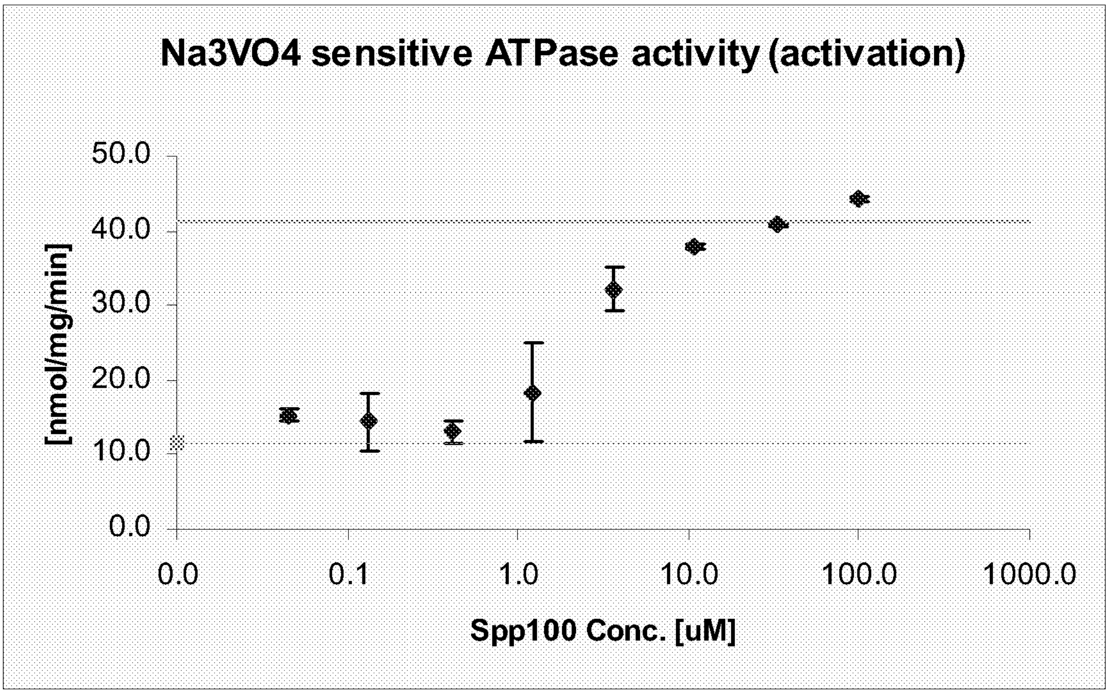
Na3VO4 sensitive ATPase activity (activation)
50.0
40.0
30.0
20.0
10.0
0.0
[nmol/mg/min]
0.0
0.1
1.0
10.0
100.0
1000.0
Spp100 Conc. [uM]

Figure 1

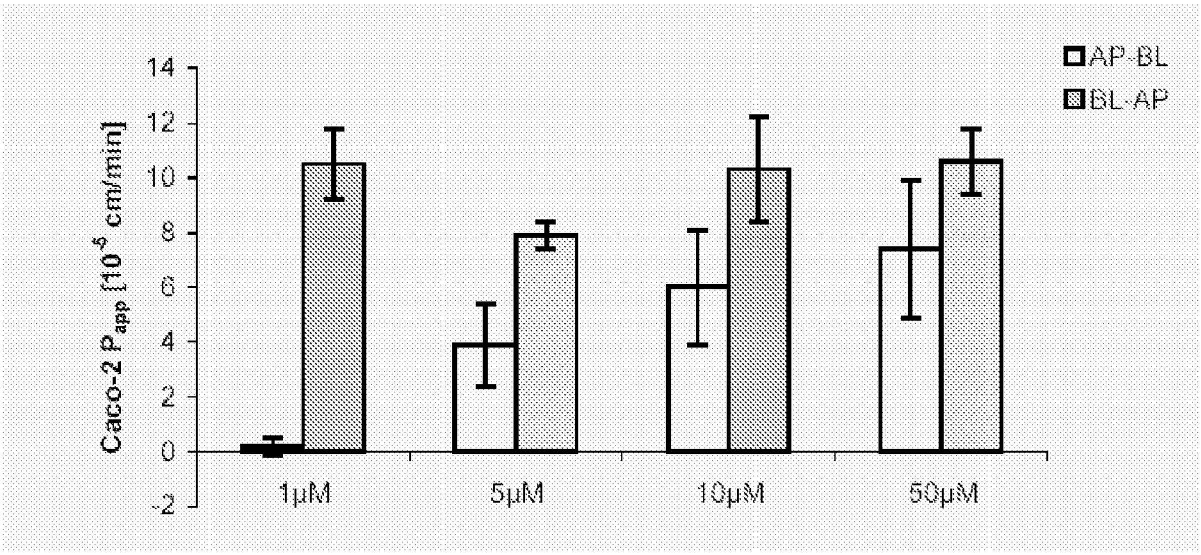


SPP100 concentration

Figure 2

European Patent Office

## PARTIAL EUROPEAN SEARCH REPORT

which under Rule 45 of the European Patent Convention shall be considered, for the purposes of subsequent proceedings, as the European search report

Application Number
EP 06 12 4774

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/058291 A (NOVARTIS AG [CH]; NOVARTIS PHARMA GMBH [AT]; OTTINGER ISABEL [DE]) 30 June 2005 (2005-06-30)<br>* abstract; claims 1-8,14,21-28; examples 10-13 *<br>* page 1, paragraphs 1,2 *<br>* page 2, paragraph 2 *<br>* page 3, paragraph 4 - page 4, paragraph 3 *<br>* page 5, paragraph 3 *<br>* page 8, paragraph 5 - page 9, paragraph 5 *<br>* page 12, paragraphs 2,3 *<br>----- | 3-8, 10-14, 16-21 | INV.<br>A61K31/165<br>A61K45/06<br>A61P9/12<br>A61P9/00 |
| Y | WO 2006/013094 A (NOVARTIS AG [CH]; NOVARTIS PHARMA GMBH [AT]; CAMENISCH GIAN P [CH]; GR) 9 February 2006 (2006-02-09)<br>* abstract; claims 1-21 *<br>----- | 3-8, 10-14, 16-21 | |
| Y | WO 03/053401 A (ALZA CORP [US]) 3 July 2003 (2003-07-03)<br>* abstract; claims 1-3,8 *<br>* paragraphs [0037], [0038], [0043] *<br>----- | 3-8, 10-14, 16-21 | TECHNICAL FIELDS SEARCHED (IPC)<br>A61K<br>A61P |

-/--

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| Munich | 18 April 2007 | GOLEBIOWSKA, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 06 12 4774

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BITTNER B ET AL: "PREFORMULATION APPROACHES TO IMPROVE THE ORAL BIOAVAILABILITY OF TWO NOVEL PIPERIDINE RENIN INHIBITORS IN DOG" ARZNEIMITTEL FORSCHUNG. DRUG RESEARCH, vol. 52, no. 8, 2002, pages 593-599, XP001207566 ISSN: 0004-4172 * abstract; table 1 * * page 594, column 1, paragraphs 1,2 * * page 595, column 1, paragraph 3 - page 595, column 2, paragraph 1 * * page 598, column 1, paragraph 1-4 * ----- | 3-8, 10-14, 16-21 | |

TECHNICAL FIELDS SEARCHED (IPC)

1

EPO FORM 1503 03.82 (P04C10)

European Patent Office

**INCOMPLETE SEARCH SHEET C**

Application Number
EP 06 12 4774

Claim(s) searched completely:
3-8, 10-14, 16-21

Claim(s) not searched:
1-2,9,15

Reason for the limitation of the search:

The pharmaceutical composition of claims 1 and 2 as well as the method of improving the bioavailability of renin of claim 9 and the use of an MDR1 inhibitor for the manufacture of a medicament of claim 15 are not clearly defined because the expression:

"an MD1 inhibitor selected from a non-pharmacologically active compound"

is vague and indefinite, thereby not allowing the skilled man to readily determine which compounds fall within the scope of this expression.

Assessing these requirements would require an equally unquantifiable and thus unreasonable amount of experimentation, imposing a severe and undue burden on all those wishing to ascertain the scope of the claim, which is not in compliance with the clarity requirement of Article 84 EPC. The non-compliance with the substantive provisions is to such an extent, that a meaningful search of the whole claimed subject-matter of the claim could not be carried out (Rule 45 EPC and Guidelines B-VIII, 3).
The search was consequently limited to exclude this possibility, and thus was restricted to:

- a pharmaceutical composition comprising a renin inhibitor and an MDR inhibitor;
- the medical use of said pharmaceutical composition;
- and to the use of MDR1 inhibitor for the manufacture of a medicament to improve the bioavailability of a renin inhibitor;

wherein the MDR1 inhibitor is selected from the group of compounds consisting of "curcumin, phenyl cinnamate, coumarin, beta-amyrin cinnamate, apiole, bergamottin, caffein, 8-(decylthio)caffeine, 8-benzylcaffein, diethylpyrocarbonate, morin, narirutin, piperine, quercetin, slavironin (salvinorin), silybin, theobromine, vanillin, vanillyl-N-nolymine (vanillyl-N-noylamide), Vitamin R TPGS, and Cremophor EL" as indicated in claims 3, 10 and 16 respectively.

## ANNEX TO THE EUROPEAN SEARCH REPORT ON EUROPEAN PATENT APPLICATION NO.

EP 06 12 4774

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2007

| Patent document cited in search report | Publication date | Patent family member(s) | Publication date |
|---|---|---|---|
| WO 2005058291 A | 30-06-2005 | AR 047287 A1 | 11-01-2006 |
| | | AU 2004298758 A1 | 30-06-2005 |
| | | BR PI0417535 A | 27-03-2007 |
| | | CA 2547195 A1 | 30-06-2005 |
| | | EP 1729748 A1 | 13-12-2006 |
| | | KR 20060130072 A | 18-12-2006 |
| | | MX PA06006926 A | 23-08-2006 |
| WO 2006013094 A | 09-02-2006 | AR 050043 A1 | 20-09-2006 |
| | | AU 2005268844 A1 | 09-02-2006 |
| | | CA 2572743 A1 | 09-02-2006 |
| WO 03053401 A | 03-07-2003 | AU 2002359793 A1 | 09-07-2003 |
| | | CA 2471096 A1 | 03-07-2003 |
| | | CN 1606432 A | 13-04-2005 |
| | | EP 1465592 A2 | 13-10-2004 |
| | | HU 0402451 A2 | 28-07-2005 |
| | | JP 2005519884 T | 07-07-2005 |
| | | MX PA04006026 A | 31-03-2005 |
| | | NZ 533060 A | 31-03-2006 |
| | | ZA 200405655 A | 15-08-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006013094 A
- US 5559111 A
- US 6197959 B
- US 6376672 B


### Non-patent literature cited in the description


- **FABER et al.** *Adv. Drug Del. Rev,* 2003, vol. 55, 107-124
- **CHAN et al.** *Eur. J. Pharmaceut. Sci.,* 2004, vol. 21, 25-51
- **BAKOS et al.** *Mol Pharmacol.,* 2002, vol. 57, 760-768
- **MAARTEN et al.** *AIDS,* 2002, vol. 16, 2295-2301
- **CAMENISCH et al.** *Pharm. Act. Helv.,* 1996, vol. 71, 309-327
- **SARKADI, B ; PRICE, E ; BOUCHER, R ; GERMANN, U ; SCARBOROUGH, G.** *J. Biol. Chem.,* 1992, vol. 267, 4854-4858
- **ARTURSSON et al.** *Biochem. Biophys. Res. Comm.,* 1991, vol. 175, 880-885